# EUROPÄISCHE PATENTANMELDUNG

(11) **EP 2 985 053 A1**
(43) Veröffentlichungstag der Anmeldung: **17.02.2016**
(21) Anmeldenummer: 15168078.2
(22) Anmeldetag: 19.05.2015
(51) Int. Cl.: A61N 1/05, A61N 1/37, A61N 1/375, A61N 1/39, H01B 7/04

(54) **IMPLANTIERBARE ELEKTRISCHE LEITUNG**

(30) Priorität: 13.08.2014 US 201462036628 P
(71) Anmelder: BIOTRONIK SE & Co. KG, 12359 Berlin (DE)
(72) Erfinder: Friedrich, Michael, 14532 Kleinmachnow (DE); Kolberg, Gernot, 12161 Berlin (DE); Frenzel, Timo, 12435 Berlin (DE)
(74) Vertreter: Galander, Marcus

(57) **Zusammenfassung**

Die Erfindung betrifft eine implantierbare elektrische Leitung (20) mit einer Zuleitung (26), die wenigstens einen helixartig gewendelten elektrischen Leiter (26.1) aufweist. Der Leiter (26.1) ist von einem Fasergeflecht umgeben, welches von mindestens zwei Fasern (50) oder Faserbündeln gebildet ist, die miteinander verflochten sind und sich in einem zueinander gegenläufigen Windungssinn um den elektrischen Leiter (26.1) winden.

## Beschreibung

Die Erfindung betrifft eine implantierbare elektrische Leitung mit einer langgestreckten elektrischen Zuleitung.

Solche Leitungen werden zum Beispiel als Elektrodenleitungen (auch kurz als "Elektroden" bezeichnet) für Herzschrittmacher oder Defibrillatoren verwendet und dienen dazu, elektrische Impulse von einem Herzschrittmacher oder Defibrillator zu Elektrodenpolen an oder nahe einem distalen Ende der Elektrodenleitung zu übertragen und umgekehrt elektrische Signale von einem oder mehreren Elektrodenpolen zum Herzschrittmacher oder Defibrillator zu übertragen. Hierzu weist eine Elektrodenleitung typischerweise eine oder mehrere elektrische Leiter aus, die eine oder mehrere Zuleitungen bilden. In implantierbaren elektrischen Leitungen wie beispielsweise Elektrodenleitungen wird vorzugsweise eine Wendel, die von einem oder mehreren helixartig gewendelten elektrischen Leitern gebildet ist, als Zuleitung eingesetzt, weil sie einerseits ein inneres Lumen für einen Führungsdraht bietet, andererseits ein gewendelter Draht deutlich stabiler gegen dauerhafte Biegebeanspruchung ist.

Der Erfindung schließt die Erkenntnis ein, dass eine Wendel als Zuleitung folgende Nachteile hat, die nach Möglichkeit zu überwinden sind:
Implantierbare Herzelektroden (das sind z.B. Elektrodenleitungen für Herzschrittmacher oder Defibrillatoren) sind unterschiedlichen mechanischen Belastungen ausgesetzt. Bei Implantation und Explantation der Elektrodenleitungen können hohe Zugkräfte auftreten. Bei ungünstiger Lage der Elektrodenleitung können radiale Drücke z. B. zwischen Schlüsselbein und erster Rippe entstehen. Solchen Kräften ist eine herkömmliche Wendel nicht gewachsen. Bei Zugkräften entdehnt sich der Wendelverbund, bei radialen Drücken wird er gestört und die Wendel ist bruchgefährdet.

Die Wendel soll einerseits weich und flexibel sein, um im Herzen keine Reaktionen zu provozieren und andererseits torsionsstabil, um einschraubbar zu sein.

Außerdem haben langgestreckte elektrische Leitungen den Nachteil, dass sich ein jeweils enthaltener elektrischer Leiter in einem Kernspintomografen erwärmen kann, weil die im Kernspintomografen herrschenden wechselnden Magnetfelder in dem elektrischen Leiter nicht unbeachtliche elektrische Ströme induzieren. Deshalb können Herzschrittmacherpatienten heutzutage in der Regel nicht oder nur eingeschränkt in einem Kernspintomografen untersucht werden.

An implantierbaren Herzschrittmachern oder Defibrillatoren sind nämlich typischerweise wenigstens eine Stimulationselektrodenleitung angeschlossen, die an ihrem proximalen, zum Anschluss an den Herzschrittmacher oder Defibrillator vorgesehenen Ende einen standardisierten elektrischen Anschluss aufweist und an ihrem distalen, zur Platzierung im Herzen vorgesehenen Ende einen oder mehrere Elektrodenpole aufweist. Ein solcher Elektrodenpol dient zur Abgabe elektrischer Impulse an das Gewebe (Myokard) des Herzens oder zum Abfühlen elektrischer Felder, um im Rahmen des sogenannten Sensings eine Aktivität eines Herzens abfühlen zu können. Zu diesen Zwecken bilden Elektrodenpole typischerweise elektrisch leitende Oberflächenabschnitte einer Elektrodenleitung. Elektrodenpole sind typischerweise als Ringelektrode in Form eines Rings um die Elektrodenleitung oder in Form einer Spitzen- oder Tippelektrode am distalen Ende der Elektrodenleitung vorgesehen. Die Elektrodenpole sind über eine oder mehrere elektrische Leiter mit Kontakten des elektrischen Anschlusses der Elektrodenleitung an deren proximalem Ende elektrisch leitend verbunden. Somit verlaufen zwischen den Kontakten des elektrischen Anschlusses die Elektrodenleitungen an deren proximalen Ende und den Elektrodenpolen am distalen Ende der Elektrodenleitung ein oder mehrere elektrische Leiter, die einen oder mehrere der Elektrodenpole mit einem oder mehreren der Kontakte elektrisch verbinden. Diese elektrischen Leiter können einerseits zur Übertragung von Stimulationsimpulsen zu den Elektrodenpolen und andererseits zur Übertragung mittels der Elektrodenpole aufgenommener elektrischer Signale zum proximalen Ende der Elektrodenleitung genutzt werden und werden im Verlauf der weiteren Beschreibung auch jeweils als Funktionsleitung bezeichnet. Solche Funktionsleitungen sind für die Funktionen der jeweiligen Elektrodenleitung erforderliche elektrische Leiter und als solche der Gefahr ausgesetzt, dass in ihnen durch äußere Wechselmagnetfelder elektrische Ströme induziert werden, die beispielsweise zu einer unerwünschten Erwärmung der Funktionsleitungen oder der mit ihr verbundenen Elektrodenpole führen können oder die zur Abgabe entsprechender Ströme über die Elektrodenpole an umgebendes Gewebe und damit zu einer Erwärmung des umgebenden Gewebes führen können.

Um eine Elektrodenleitung weniger empfindlich für MRI-Energien zu machen, ist es sinnvoll, die Wendel mit einer hohen Induktivität auszustatten. Diese wird mit möglichst vielen Windungen erreicht, was die Wendel wiederum mechanisch instabil machen würde.

Die mechanischen Nachteile einer Wendel werden bei bekannten Elektrodenleitungen durch äußeren Schutz kompensiert. Es werden möglichst stabile Außenschläuche zur elektrischen Isolation und zum mechanischen Schutz eingesetzt. Dabei werden im Katheterbereich häufig Geflechtsschläuche eingesetzt, um den Katheter knick- und torsionsstabil zu gestalten. Im Elektrodenbereich werden solche Konzepte bisher nicht eingesetzt. Elektroden, deren Wendeln durch Seile ersetzt wurden, haben kein Lumen für den Führungsdraht und werden mit speziellen Kathetern implantiert.

Sonst müssen die Wendelgeometrien in den geometrischen Dimensionen gehalten werden, die dem Einsatz entsprechend stabil sind.

Um eine Elektrode unempfindlich gegen die hochfrequenten Energien aus dem MRT zu machen, werden zusätzliche Bauelemente wie induktive Filter, Bandstoppfilter oder zusätzliche Energieableiter eingesetzt.

Der Erfindung liegt die Aufgabe zugrunde, eine implantierbare elektrische Leitung zu schaffen, welche möglichst weniger Nachteile als bekannte Elektrodenleitungen besitzt. Erfindungsgemäß wird diese Aufgabe durch eine implantierbare elektrische Leitung gelöst, welche eine Zuleitung aufweist, die wenigstens einen helixartig gewendelten elektrischen Leiter aufweist, wobei der Leiter von einem Fasergeflecht umgeben ist, welches von mindestens zwei Fasern oder Faserbündeln gebildet ist, die miteinander verflochten sind und sich in einem zueinander gegenläufigen Windungssinn um den elektrischen Leiter winden.

Die implantierbare elektrische Leitung enthält im Ergebnis eine neuartige Zuleitungskonstruktion, die viele Nachteile der bekannten Wendel überwindet.

Das zugrundeliegende Konzept ist, eine Wendel unmittelbar mit einem Geflecht zu kombinieren. Wendel und Geflecht werden zu einer mechanischen Einheit. Gemäß diesem Konzept ist elektrische Zuleitung für eine implantierbare elektrische Leitung vorgesehen, wobei die Zuleitung von mindestens einem Draht und mindestens zwei Fasern oder Faserbündel gebildet wird, von denen ein oder mehrere Drähte zu einer Wendel gewickelt sind und mindestens zwei Fasern oder Faserbündel um diese Wendel geflochten und mindestens an einem Ende mit der Wendel mechanisch verbunden sind.

Durch dieses Konzept werden folgende Eigenschaften der elektrischen Leitung verbessert:
Zugstabilität; Torsionssteifigkeit, radiale Druckstabilität; Knickstabilität, Verringerung der HF-Übertragungseigenschaften und in bestimmten Ausführungen auch der elektrische Widerstand.

Die gewünschte Flexibilität und Dauerstabilität der Zuleitung bleibt weitgehend erhalten. Durch die erfindungsgemäße Konstruktion können neue Wendelgeometrien realisiert werden, die bisher zu instabil waren (dünne Wendeldrähte, Einfachwendeln, Wendeln mit hoher Induktivität etc).

Außerdem können mit der erfindungsgemäßen elektrischen Leitung Zuleitungen mit einem Innenlumen realisiert werden, die eine hohe Flexibilität und eine hohe Dauerlastfestigkeit aufweisen und gleichzeitig wenigstens einige der folgenden Nachteile zumindest teilweise überwindet:
- Mangelhafte Zugfestigkeit
- Schlechte radiale Druckstabilität
- Schlechte Torsionsübertragung
- Schlechte HF-Dämpfung

Der Erfindung schließt außerdem die Erkenntnis ein, dass bekannte Wendeln als Zuleitungen folgende Nachteile aufweisen:

Eine Wendel, die mechanisch durch ihre Isolation geschützt werden muss, schränkt die Designmöglichkeiten eines Produktes ein.

Eine Wendel, die widersprüchliche mechanische oder elektrische Eigenschaften hat, ist in beide Richtungen immer ein Kompromiss, der zu verbesserungswürdigen Eigenschaften des Produktes führt. Ein Geflechtschlauch, der die Isolation verstärkt, ist relativ steif und kann die Torsionseigenschaften der Wendel selbst nicht verbessern.

Zusätzliche Bauelemente in einer Elektrode, die die MR-Eigenschaften verbessern sollen, haben immer mechanische Nachteile. Eine Elektrode kann dadurch steifer werden oder dicker. Die Dauerfestigkeit der Elektrode kann insbesondere dann beeinträchtigt werden, wenn elektrisch wirksame Komponenten in einer Reihenschaltung in den therapeutischen Kreis integriert werden.

Vorzugsweise bildet der wenigstens eine helixartig gewendelte elektrische Leiter eine Wendel, die an einem oder an beiden Längsenden mit einer elektrisch leitenden Hülse elektrisch leitend und mechanisch verbunden ist, wobei die Fasern mit der und/oder den elektrisch leitenden Hülsen mechanisch verbunden sind.

Vorzugsweise bildet wenigstens eine der Hülsen mit einer zu dieser konzentrischen, von der Hülse durch ein Dielektrikum elektrisch isolierten Wendelhülse eine Kapazität. Vorzugsweise ist der wenigstens eine helixartig gewendelte elektrische Leiter von einem Draht aus einem oder mehreren der folgenden Metalle gebildet: Pt, Pt/Ir, MP35N, Elgiloy, Edelstahl, Kupfer, Molybdän, Tantal, Gold, Zirkonium, eisenhaltige Legierungen, Tantal-Wolframlegierungen, Refraktaermetalle wie Niob oder Titan, Silber, Palladium, leitfähige Polymere oder Polykondensate oder Kombinationen in einem Mantel/Kern-Draht.

Vorzugsweise sind die Fasern aus einem oder mehreren der folgenden Metalle oder Kunststoffe gebildet: Pt, Pt/Ir, MP35N, Elgiloy, Edelstahl, Kupfer, Molybdän, Tantal, Gold, Zirkonium, eisenhaltige Legierungen, Tantal-Wolframlegierungen, Refraktaermetalle wie Niob oder Titan, Silber, Palladium, leitfähige Polymere oder Polykondensate oder Kombinationen in einem Mantel/Kern-Draht sowie Peek, Polyamid, Polyurethan, Carbonfasern, Polyethylen, Polyimid, Aramid, PTFE, ETFE, Spinnengewebe, Spinnenfasern, künstliche Spinnenseide.

Der wenigstens eine helixartig gewendelte elektrische Leiter ist vorzugsweise durch eine Beschichtung isoliert.

Alternativ oder zusätzlich können auch die Fasern durch eine Beschichtung isoliert sein.

Vorzugsweise ist zwischen dem wenigstens einen helixartig gewendelten elektrischen Leiter und den Fasern eine Isolation, z.B. ein isolierender Schlauch, angeordnet.

Vorzugsweise enthält das von den Fasern gebildete Fasergeflecht sowohl elektrisch nicht-leitende Fasern aus Kunststoff als auch elektrisch leitenden Fasern aus Metall.

Die implantierbare elektrische Leitung kann eine Zuleitung aufweisen, die nur in einem oder mehreren Abschnitten erfindungsgemäß ausgebildet ist.

Die implantierbare elektrische Leitung ist vorzugsweise eine Herzschrittmacher- und/oder Defibrillator-Elektrodenleitung und weist einen oder mehrere Elektrodenpole auf, die mit der Zuleitung elektrisch verbunden sind.

Vorzugsweise ist die Zuleitung so ausgebildet, dass der wenigstens eine helixartig gewendelte elektrische Leiter und/oder das Fasergeflecht alleine oder in Verbindung miteinander Induktivitäten und/oder Kapazitäten bilden, die filternde Eigenschaften haben und/oder einen Filter bilden, die bei Frequenzen der in Kernspintomografen herrschenden elektromagnetischen Felder dämpft. eine Elektrodenleitung mit einer derartigen Zuleitung weist im Ergebnis nicht nur vorteilhafte mechanische Eigenschaften auf, sondern ist darüber hinaus auch noch für einen Einsatz in einem Kernspintomografen tauglich.

Folgende weitere Ausführungsvarianten sind vorteilhaft:
- Der die Wendel bildende elektrische Leiter ist ein Flachdraht.
- Die Fasern des Fasergeflechts haben einen Durchmesser zwischen 0,005 und 0,2 mm
- Zwischen den Fasern des Fasergeflechts und der Wendel befindet sich isolierender Schlauch oder ein isolierendes zusätzliches Geflecht.
- Die Fasern des Fasergeflechts sind an beiden Enden elektrisch mit der Wendel verbunden.
- Die Fasern des Fasergeflechts sind nur einseitig mit der Wendel verbunden.
- Die Fasern des Fasergeflechts sind von der Wendel elektrisch vollständig isoliert.
- Die Fasern des Fasergeflechts sind beidseitig kapazitiv mit dem wenigstens einen helixartig gewendelte elektrische Leiter verbunden.
- Die Fasern des Fasergeflechts sind an einem Ende kapazitiv mit dem wenigstens einen helixartig gewendelte elektrische Leiter verbunden und am anderen Ende galvanisch.
- Die Fasern des Fasergeflechts sind an einem Ende kapazitiv mit dem wenigstens einen helixartig gewendelte elektrische Leiter verbunden und am anderen Ende elektrisch offen (hochohmig).
- Die Fasern des Fasergeflechts sind auf einen Schlauch geflochten und mit einer Wendel umgeben und mit dieser an beiden Enden mechanisch verbunden.
- Die Zuleitung einer Elektrode besteht aus mehreren erfindungsgemäß konstruierten Segmenten, die vorzugsweise hintereinander geschaltet sind.
- Bei einer Reihenschaltung mehrerer Zuleitungssegmente wechselt der Wickelsinn der Wendel des jeweiligen Zuleitungssegmentes von einem zum anderen Zuleitungssegment. Dadurch wird das Dämpfungsverhalten beeinflusst.
- Die Wendel ist von einem zwei oder mehrlagigen Fasergeflecht umgeben.
- Sowohl der wenigstens eine helixartig gewendelte elektrische Leiter als auch die Fasern des Fasergeflechts können eine Beschichtung aus Peek, Polyamid, Polyurethan, Carbonfasern, Polyethylen, Polyimid, Aramid, PTFE und/oder ETFE haben.
- Bestehende Zuleitungskonzepte (z. B. eine konventionelle Wendel) werden mit einem oder mehreren erfindungsgemäß konstruierten Konzepten verbunden (Reihenschaltung).

Die Erfindung soll nun anhand von Ausführungsbeispielen mit Bezug auf die Figuren näher erläutert werden. Die Figuren zeigen Folgendes:
- Fig. 1: zeigt einen implantierbaren Herzstimulator 10 und eine daran angeschlossene implantierbare Elektrodenleitung 20.
- Fig. 2: zeigt eine konventionelle Wendel, deren Enden mit Hülsen verschweißt sind.
- Fig. 3: zeigt ein elektrisches Ersatzschaltbild für die Wendel aus Fig. 2.
- Fig. 4: zeigt eine Wendel ähnlich der aus Fig. 2 nachdem die Wendel mit einer Vielzahl dünner Einzeldrähte umflochten wurde.
- Fig. 5: zeigt ein elektrisches Ersatzschaltbild für die Wendel aus Fig. 4.
- Fig. 6: zeigt eine Zuleitung, die von zwei kurzen, hintereinander geschalteten, erfindungsgemäßen Zuleitungssegmenten gebildet ist.
- Fig. 7: zeigt eine Elektrodenleitung, deren Außenleiter von einer umflochtenen Außenwendel gebildet ist.
- Fig. 8: zeigt eine Elektrodenleitung, deren Innenleiter von einer Kombination aus bekannter Wendel als Zuleitung und erfindungsgemäßem Zuleitungskonzept gebildet ist.
- Fig. 9: zeigt ein distales Innenleitersegment, das von einer umflochtenen Wendel gebildet ist, deren Geflecht auf der linken Seite mit einer Hülse verschweißt ist, die zuvor auf die isolierend beschichtete Wendelhülse gehämmert wurde.
- Fig. 10: zeigt das resultierende elektrische Ersatzschaltbild für die Konstruktion von Fig. 9.
- Fig. 11: zeigt ein elektrisches Ersatzschaltbild einer weiteren Ausführungsvariante der erfindungsgemäßen Zuleitung.

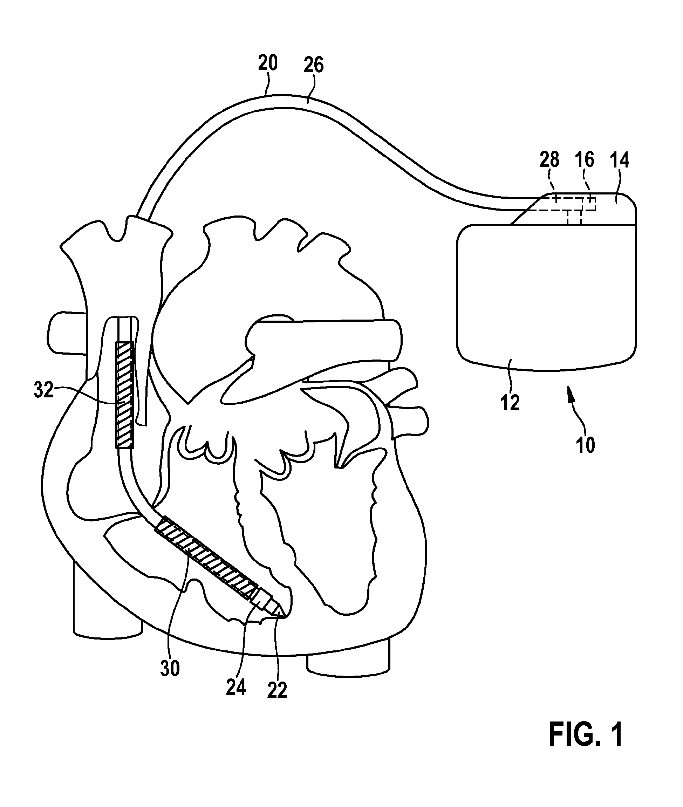

Fig. 1 zeigt einen implantierbaren Herzstimulator 10 und eine daran angeschlossene implantierbare Elektrodenleitung 20. Der implantierbare Herzstimulator 10 kann ein Herzschrittmacher oder ein Kardioverter/Defibrillator (ICD) sein. Im dargestellten Ausführungsbeispiel ist der Herzstimulator 10 ein ventrikulärer Herzschrittmacher und Defibrillator. Andere bekannte Herzstimulatoren sind Zweikammerherzschrittmacher zur Stimulation des rechten Atriums und des rechten Ventrikels oder biventrikuläre Herzschrittmacher, die zusätzlich zum rechten Ventrikel auch den linken Ventrikel stimulieren können.

Derartige Stimulatoren besitzen typischerweise ein Gehäuse 12, das im Regelfall aus Metall besteht und somit elektrisch leitend ist und als großflächiger Elektrodenpol dienen kann. An der Außenseite des Gehäuses 12 ist typischerweise ein Anschlussgehäuse 14 befestigt, das auch als Header bezeichnet wird. Ein derartiger Header weist typischerweise Kontaktbuchsen zur Aufnahme von Steckkontakten auf. Die Kontaktbuchsen besitzen elektrische Kontakte 16, die über entsprechende Leiter mit einer in dem Gehäuse 12 des Herzstimulators 10 angeordneten Elektronik verbunden sind.

Die Elektrodenleitung 20 ist eine implantierbare elektrische Leitung. Am distalen Ende der Elektrodenleitung 20 sind in an sich bekannter Manier Elektrodenpole in Form einer Spitzen- oder Tipelektrode 22 und einer in deren Nähe angeordneten Ringelektrode 24 angeordnet. Die Elektrodenpole 22 und 24 sind so ausgebildet, dass sie je nach Funktion eines Herzstimulators, an den die Elektrodenleitung 20 angeschlossen ist, zum Abfühlen elektrischer Potenziale des Herzgewebes (Myokards) dienen oder zur Abgabe elektrischer Signale, beispielsweise zur Abgabe von Stimulationsimpulsen an das sie umgebende Herzgewebe, ausgebildet sind. Fig. 1 zeigt, wie sich die Elektrodenpole, also die Tipelektrode 22 und die Ringelektrode 24, im Anwendungsfall die Elektrodenleitung 20, im Apex eines rechten Ventrikels eines Herzens befinden.

Sowohl die Tipelektrode 22 als auch die Ringelektrode 24 sind über jeweils wenigstens einen elektrischen Leiter 26.1 und 26.2 mit einem Steckkontakt 28 am proximalen Ende der Elektrodenleitung 20 elektrisch verbunden. Der Steckkontakt 28 besitzt elektrische Kontakte, die mit den elektrischen Kontakten 16 der Kontaktbuchse im Anschlussgehäuse 14 des implantierbaren Herzstimulators korrespondieren. Die elektrischen Leiter 26.1 und 26.2 in der Elektrodenleitung 20 sind zu einer gemeinsamen Helix gewendelt und bilden so eine Zuleitung 26 in Form einer Coradialwendelzuleitung mit einer koradialen Drahtwendel, die von zwei zu jeweils einer Helix mit identischem Durchmesser und Steigung gewickelten Drähten gebildet ist. Anstelle einer Coradialwendelzuleitung können auch mehrere zueinander coaxiale Wendeln mit jeweils unterschiedlichem Durchmesser vorgesehen sein. In diesem Fall ergibt sich eine Coaxialwendelzuleitung. Neben den der Signalübertragung dienenden Leiter 26.1 und 26.2, die auch als Funktionsleiter bezeichnet werden, kann eine jeweilige Zuleitung auch weitere Leiter, z.B. Drähte, die die Struktur der Zuleitung unterstützen, aufweisen.

Die von den elektrischen Leitern 26.1 und 26.2 gebildete Zuleitung 26, die die Elektrodenpole 22 bzw. 24 mit den elektrischen Kontakten des Steckers 28 der Elektrodenleitung 20 verbinden, sind über den größten Teil ihrer Länge von einer isolierenden Hülle umgeben, so dass ein elektrischer Kontakt zum Gewebe des Herzens gezielt über die Elektrodenpole zustande kommt.

Neben den Elektrodenpolen 22 und 24, die typischerweise der (in diesem Fall ventrikulären) Stimulation des Herzgewebes dienen, weist die Elektrodenleitung 20 auch noch zwei großflächigere Elektrodenpole 30 und 32 auf, die als Defibrillationselektroden dienen und von wenigstens einem blank liegendem helixartig gewendelten Draht gebildet sind.

Es sei darauf hingewiesen, dass die Erfindung im Rahmen dieses Ausführungsbeispiels anhand eines rechtsventrikulären Herzschrittmachers und Defibrillators erläutert wird. Als medizinisches Gerät im Sinne der Erfindung kann grundsätzlich aber beispielsweise auch eine Ablationselektrodenleitung dienen, die im Anwendungsfall ebenfalls bis ins Herz eines Patienten hineinragt und die von einem außerhalb des Patienten angeordneten Gerät gesteuert wird und hierzu mit diesem verbunden ist.

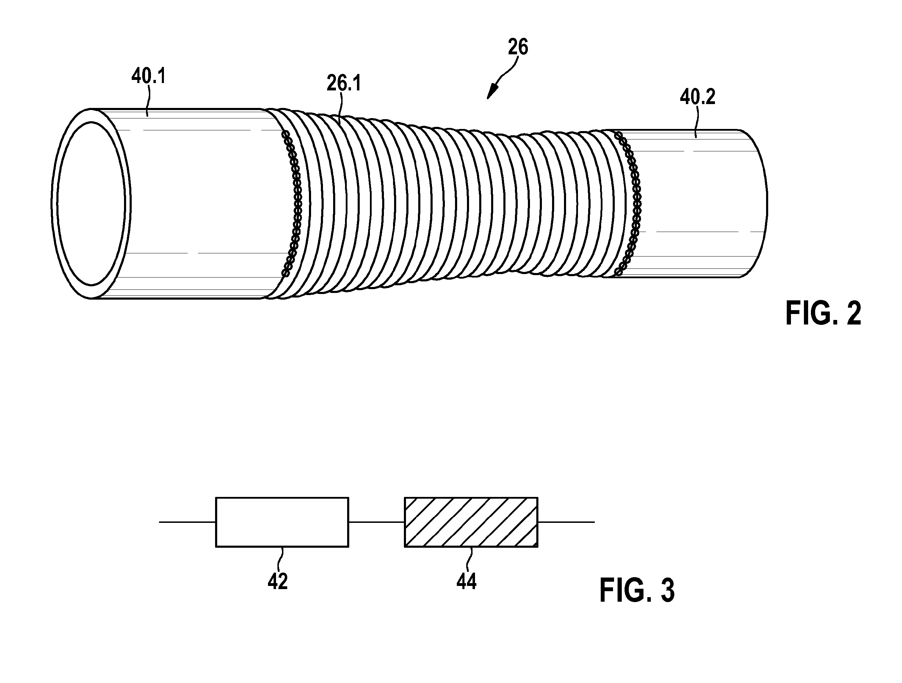

Fig. 2 zeigt eine konventionelle Wendel 26 einer Zuleitung, die von einem helixförmig gewendelten Draht 26.1 gebildet ist. Die Längsenden der Wendel sind mit Hülsen 40.1 und 40.2 verschweißt. Wendeln dieser Art werden z. B. für Innenleiter bei koaxial aufgebauten Elektrodenleitungen eingesetzt. Mit ihnen wird die Torsion zur Bedienung des Schraubmechanismus' übertragen. Das elektrische Ersatzschaltbild für die Wendel aus Fig. 2 ist eine Reihenschaltung aus Widerstand 42 und Induktivität 44; siehe Fig. 3.

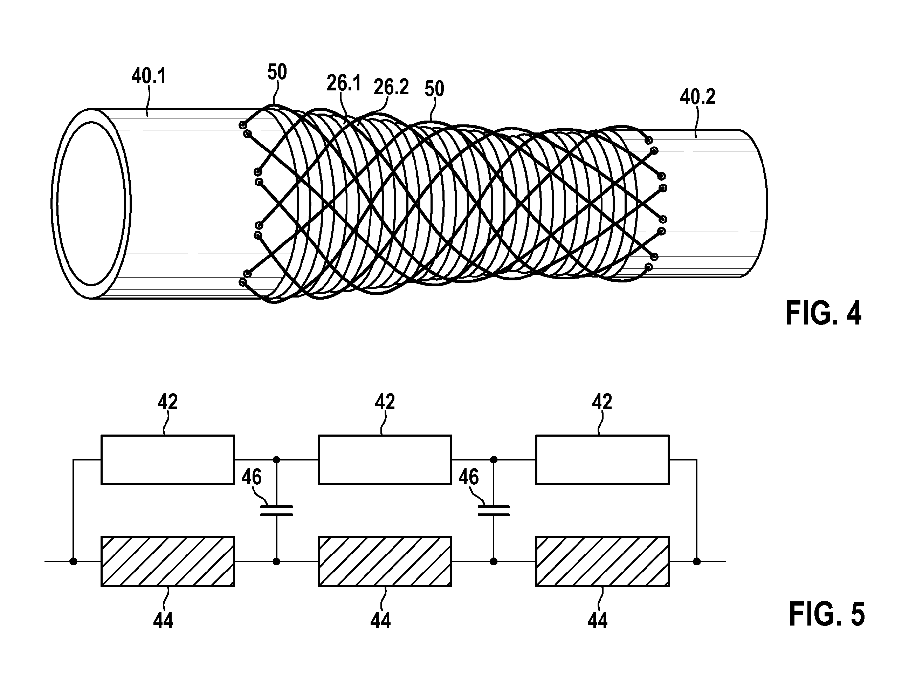

Fig. 4 zeigt eine Wendel 26 ähnlich der aus Fig. 2 nachdem die Wendel 26 mit einer Vielzahl dünner Einzeldrähte 50 umflochten wurde und so eine erfindungsgemäße Zuleitung bildet mit helixartig gewendeltem elektrischen Leiter 26.1, der die Wendel 26 bildet, und einem diese umgebenden Fasergeflecht aus Fasern 50. Eine solche Zuleitung ist mechanisch sehr stabil und eignet sich als Innenleiter in diversen Elektrodenleitungskonstruktionen. Die Zuleitung überträgt Drehmomente zum Ausschrauben einer Fixierung. Sie überträgt sicher Zugkräfte für den Fall, dass die Elektrodenleitung, deren Teil sie ist, explantiert werden muss.

Jeder der Einzeldrähte 50 kann aus Metall gefertigt sein und ist dann ein Leiter oder Teil eines Leiters. Anstelle von oder zusätzlich zu Einzeldrähten aus Metall können auch Fasern oder Filamente aus anderen Materialen, insbesondere Kunststoff, vorgesehen sein. Daher werden im Rahmen dieser Beschreibung auch Einzeldrähte aus Metall allgemein als "Fasern" bezeichnet.

Geeignete Materialien für den die Wendel der Zuleitung bildenden Metalldraht 26.1 sind: Pt, Pt/Ir, MP35N, Elgiloy, Edelstahl, Kupfer, Molybdän, Tantal, Gold, Zirkonium, Tantal-Wolframlegierungen, Refraktaermetalle wie Niob oder Titan, Palladium, leitfähige Polymere oder Polykondensate oder Kombinationen in einem Mantel/Kern-Draht, eisenhaltige Legierungen, Silber oder Kombinationen in einem Mantel/Kern-Draht.

Geeignete Materialien für die Fasern 50 des Fasergeflechts sind Metalle, wie sie auch für die Wendel der Zuleitung geeignet sind (s.o.) und außerdem Kunststoffe wie Peek, Polyamid, Polyurethan, Carbonfasern, Polyethylen, Polyimid, Aramid, PTFE, ETFE etc.

Ein jeweiliger, die Wendel bildender Metalldraht 26.1 hat vorzugsweise einen Drahtdurchmesser zwischen 0,03 und 0,2 mm. Ist die Wendel 26 eine Coradialwendel und dementsprechend von mehreren Metalldrähten 26.1 gebildet, können die die Wendel bildenden Drähte aus verschiedenen Metallen gefertigt sein, bestehen jedoch i.d.R. auch aus demselben Material wie die übrigen, die Coradialwendel bildenden Drähte.

Der jeweilige Metalldraht 26.1 der Wendel 26 ist vorzugsweise ein Flachdraht.

Die Fasern 50 des Fasergeflechts haben vorzugsweise einen Durchmesser zwischen 0,005 und 0,2 mm.

Falls die Fasern 50 von elektrisch leitenden Drähten gebildet sind, kann es dort, wo sich die von den Einzeldrähten 50 gebildeten Leiter an den Kreuzungspunkten zur Wendel sehr dicht gegenüber liegen, zu einer kapazitiven Kopplung kommen. Ein elektrisches Ersatzschaltbild kann dann so aussehen, wie es in Fig. 5 dargestellt ist und enthält neben ohmschen Widerständen 42 und Induktivitäten 44 auch Kapazitäten 46.

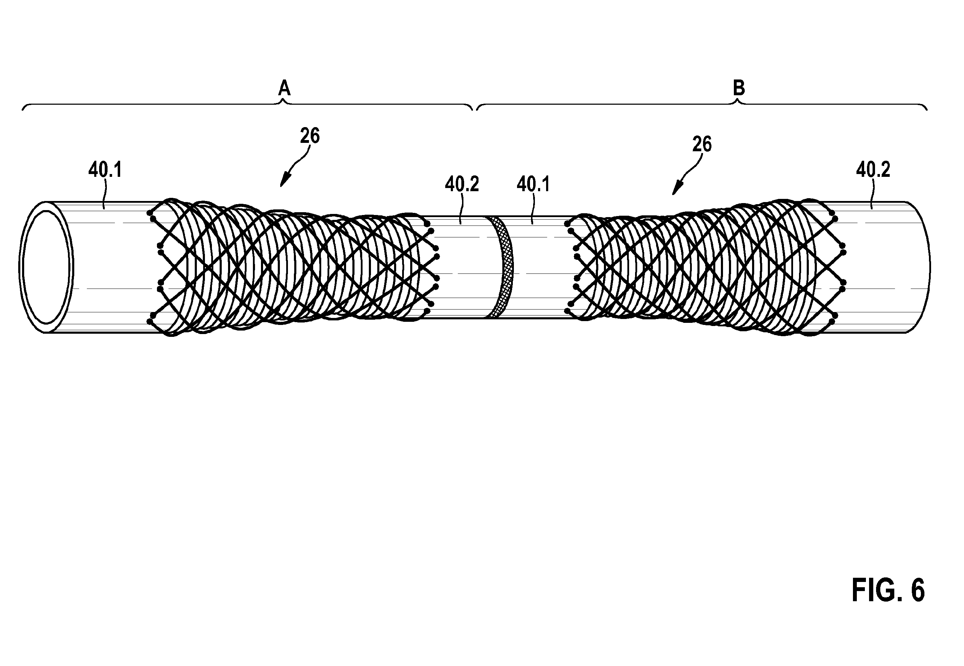

Fig. 6 zeigt zwei kurze Zuleitungssegmente A und B, die nach Art der in Fig. 3 gezeigten Zuleitungs-Konstruktion aufgebaut und die hintereinander geschaltet sind, wobei der Windungssinn der beiden Wendeln der hintereinander geschalteten Zuleitungssegmenten einander entgegengesetzt ist.

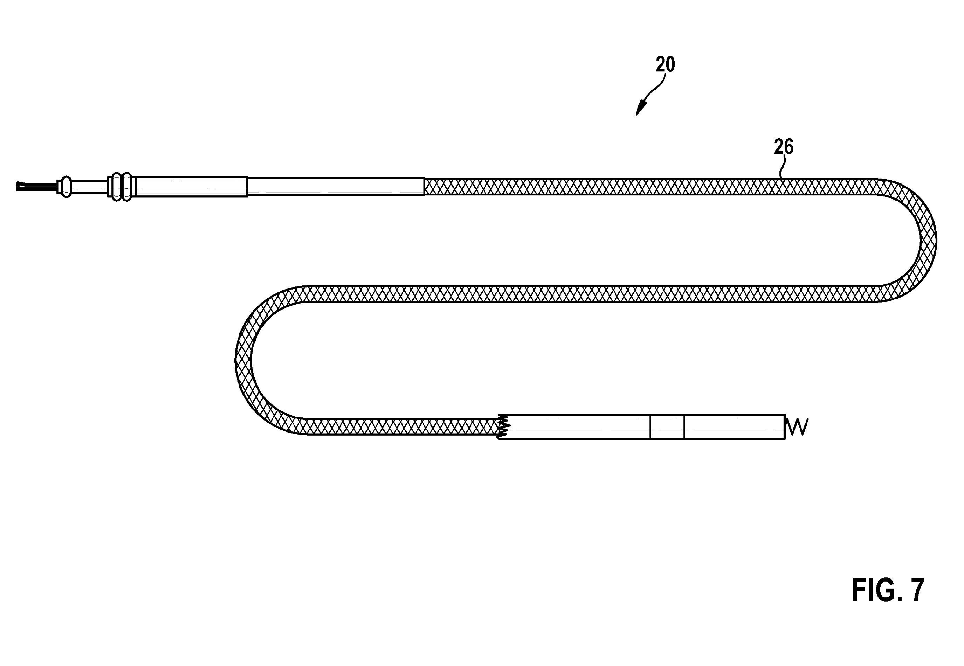

Fig. 7 zeigt eine Elektrodenleitung 20, deren Außenleiter von einer umflochtenen Außenwendel 26 gebildet ist. Diese Elektrodenleitung 20 ist besonders resistent gegen subclavian crush. Gleichzeitig kann bei dieser Elektrodenleitung auch ein jeweiliger Innenleiter umflochten sein. So kann ein Drehmoment besonders gut zwischen Kopf und ausschraubbarer Fixierhelix übertragen werden.

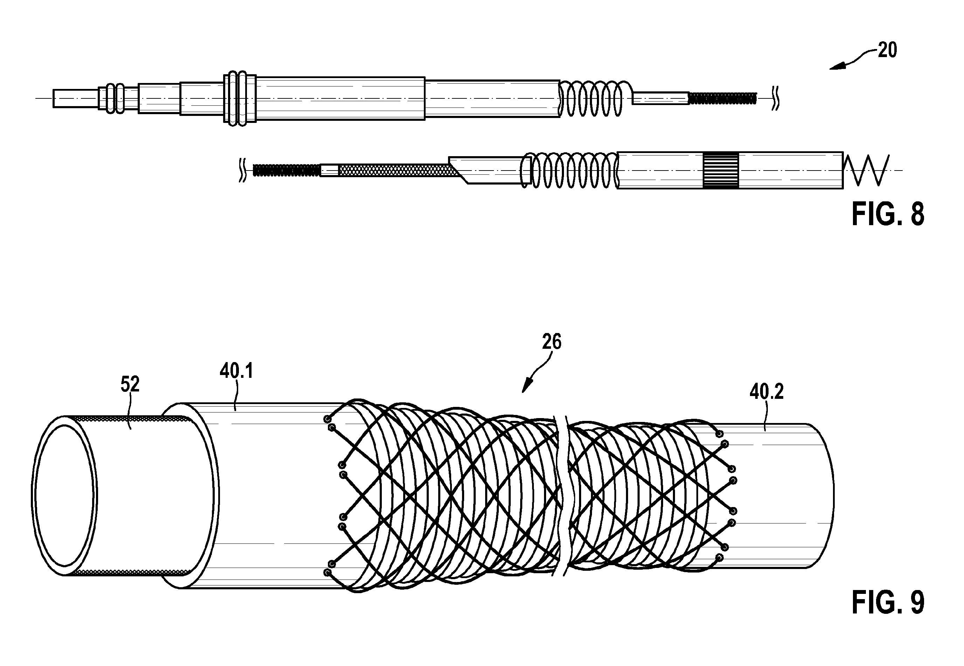

Fig. 8 zeigt eine Elektrodenleitung 20, deren Innenleiter von einer Kombination aus bekannter Wendel als Zuleitung und erfindungsgemäßem Zuleitungskonzept gebildet ist. Die Wendel besteht aus 4 parallel liegenden Einzelfilamenten, als gewendelten Leitern 26.1, 26.2, 26.3 und 26.4 aus Draht, die einen niedrigen Gesamtwiderstand der Zuleitung ermöglichen. Im distalen Bereich der Zuleitung befindet sich ein kurzes umflochtenes Zuleitungssegment. Dieses Segment wird in Fig. 9 beschrieben.

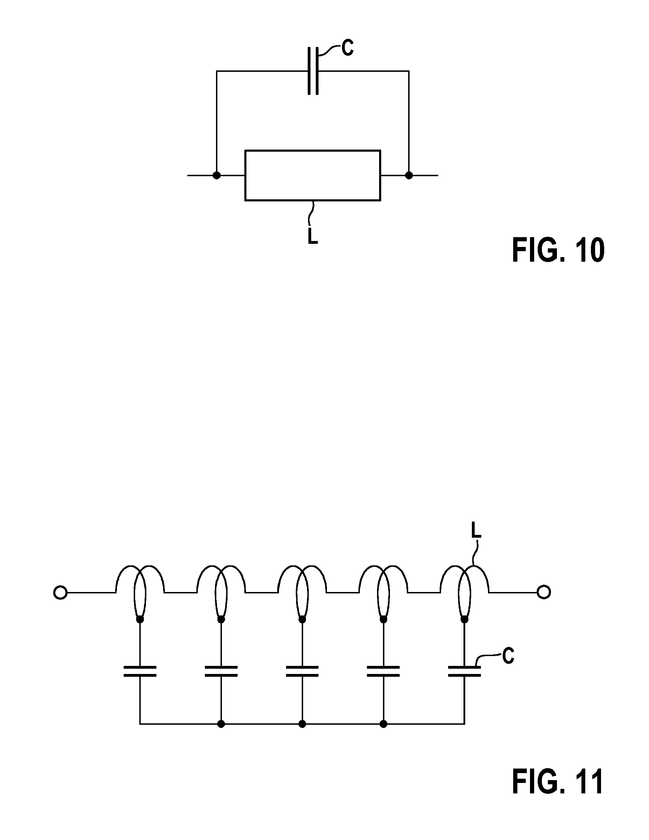

Fig. 9 zeigt ein distales Innenleitersegment, das von einer umflochtenen Wendel gebildet ist, deren Geflecht auf der linken Seite mit einer Hülse 40.1 verschweißt ist, die zuvor auf eine isolierend beschichtete Wendelhülse 52 gehämmert wurde, so dass eine elektrische Kapazität zwischen der inneren Wendelhülse 52 und der äußeren Hülse 40.1 entsteht, also ein Kondensator gebildet wird. Der den Leiter 26.1 bildende Wendeldraht ist elektrisch isolierend beschichtet. So ist das von den Fasern 50 gebildete Geflecht einseitig kapazitiv von der Wendel 26 getrennt. Am gegenüberliegenden Ende jedoch ist es mit der Wendel 26 verbunden. Die in diesem Segment eingesetzte Wendel enthält nur 2 parallele Wendelfilamente, die etwas dünner sind als er Wendeldraht 26.1 der Zuleitungswendel 26 (Fig. 8). So wird die Induktivität dieses Zuleitungssegmentes gegenüber einer normalen Wendel deutlich erhöht. Die kapazitiven Eigenschaften der dielektrisch beschichteten Wendelhülse 52 und die induktiven Eigenschaften der Wendelkomponenten sind so aufeinander abgestimmt, dass ein Ersatzschaltbild gem. Fig. 10 entsteht.

Fig. 10 zeigt das resultierende elektrische Schaltbild für die Konstruktion von Fig. 9. Das Wendelsegment bildet eine Induktivität L, zu dem eine Kapazität C parallel geschaltet wird; Die Charakteristik dieser Konstruktion entspricht der eines Bandstoppfilters. Die Kapazität von C wird so abgestimmt oder gewählt, dass die Resonanz des Filters eine gute HF-dämpfende Wirkung nahe Anregungsfrequenzen der HF-Antenne des Kernspintomografen ermöglicht. Bei einem 1,5 T Kernspintomografen ist diese beispielsweise ca. 64 MHz. In einem realen Filter besitzen beide Komponenten - Wendel und Fasergeflecht - tatsächlich induktive, kapazitive und resistive Anteile.

Bei einer Reihenschaltung mehrerer solcher Elemente können alle Elemente dieselbe Resonanzfrequenz besitzen, um die Gesamt-Filterwirkung zu verstärken, oder einzelne Elemente können für andere Frequenzen (z.B. die eines 3 T Kernspintomografen, ca. 128 MHz) gestimmt sein.

Weitere mögliche Ausführungsformen eines geeigneten Kondensators sind:
(a) Wie in Fig. 9: Eine zuvor aufgetragene dielektrische Schicht ist zwischen zwei konzentrischen Hülsen an einem Ende einer Zuleitung oder eines Zuleitungssegmentes vorgesehen. Geeignete dielektrische Schichten können von folgenden Materialien gebildet sein: Siliziumcarbid, Aluminiumoxid, oxidierte Oberflächen der Hülsen, Parylene, Polyimid, Polyethylene.
(b) Wie in Fig. 9, aber mit einem von zwei voneinander isolierten, konzentrischen Hülsen gebildete Kapazität in beiden Endhülsen.
(c) Alternativ oder zusätzlich zu von Hülsen gebildeten Kapazitäten kann eine Kapazität zwischen den Flechtfaser /-bündeln gebildet sein. So kann z.B. nur die Hälfte der Flechtfasern /-bündel mit einer Endhülse galvanisch, und am anderen Ende mechanisch aber nicht galvanisch kontaktiert sein. Die andere Hälfte der Flechtfasern /-bündel(die von den anderen z.B. durch eine dielektrische Beschichtung getrennt sind) wird dementsprechend am gegenüberliegenden Ende kontaktiert.
(d) Alternativ oder zusätzlich kann eine Kapazität zwischen den - isolierten -- Flechtfaser/- bündeln (die nur mit einer Endhülse galvanisch kontaktiert sind) und einer außen die Fasergeflechtisolierung aufgebrachte, elektrisch leitende (z.B. Metall-) Schicht (die mit der anderen Endhülse kontaktiert ist) gebildet sein.
(e) Alternativ oder zusätzlich können die Flechtfasern 50 oder entsprechenden Faserbündel mit einem oder beiden der Endhülsen nicht galvanisch kontaktiert sein, sondern dienen der kapazitiven Kopplung der einzelnen Windungen entlang der Wendel. Somit ist das Geflecht aus den Fasern 50 entweder elektrisch flotierend oder nur einseitig mit der Wendel 26 kontaktiert. Die so entstehende, verteilte Kapazität führt dann zur gewünschten Filterresonanz. Das elektrische Schaltbild, Fig. 11, lässt sich zu äquivalenten, diskreten Komponenten L und C wie in Fig. 10 zusammenfassen.

Die konzentrischen Hülsen der Kondensator-Ausführungsvariante (a) könnten folgendermaßen zusammengefügt werden:
(1) wie in Fig. 9: entweder eine Innen- oder eine Außenhülse wird zuvor dielektrisch beschichtet, dann werden die Hülsen ineinander geschoben und zusammengehämmert.
(2) Wie (1) aber die Hülsen werden durch verschiedene thermische Dehnung zusammengefügt, d.h.: Innenhülse wird gekühlt, Außenhülse wird erwärmt, Hülsen werden ineinander geschoben und bilden nach Temperaturangleichung dann eine feste Verbindung (Presspassung).
(3) Die Hülsen sind zuvor unbeschichtet. Sie werden ineinandergeschoben (lockere Passung) und in eine Elektrolytlösung getaucht, dann wird von einer Hülse zur anderen Strom durchgeführt, womit durch galvanische Oxidation eine Schicht zwischen den Hülsen gebildet wird. Diese Schicht dient dann als Dielektrikum. Geeignete Materialien für mindestens eine dieser Hülsen sind z.B. die, die sich anodisieren lassen, z.B. Titan, Tantal, Niob oder Aluminium.

### Bezugszeichenliste

- 10: Implantierbarer Herzstimulator
- 12: Gehäuse
- 14: Anschlussgehäuse
- 16: Kontakte
- 20: Elektrodenleitung
- 22: Tipelektrode
- 24: Ringelektrode
- 26.1, 26.2: Elektrische Leiter
- 26: (Wendel-) Zuleitung
- 28: Steckkontakt
- 30, 32: Elektrodenpol
- 40.1, 40.2: Hülse
- 42: Ohmscher Widerstand
- 44: Induktivität
- 46: Kapazität
- 50: (Draht-) Faser
- 52: Wendelhülse

## Patentansprüche

1. Implantierbare elektrische Leitung (20) mit einer Zuleitung (26) oder einem Zuleitungssegment, die oder das wenigstens einen helixartig gewendelten elektrischen Leiter (26.1) aufweist, **dadurch gekennzeichnet, dass** der Leiter (26.1) von einem Fasergeflecht umgeben ist, welches von mindestens zwei Fasern (50) oder Faserbündeln gebildet ist, die miteinander verflochten sind und sich in einem zueinander gegenläufigen Windungssinn um den elektrischen Leiter (26.1) winden.

2. Implantierbare elektrische Leitung (20) gemäß Anspruch 1, **dadurch gekennzeichnet, dass** der wenigstens eine helixartig gewendelte elektrische Leiter (26.1) eine Wendel (26) bildet, die an einem oder an beiden Längsenden mit einer elektrisch leitenden Hülse (40.1, 40.2) elektrisch leitend und mechanisch verbunden ist und dass die Fasern (50) mit der und/oder den elektrisch leitenden Hülsen (40.1, 40.2) verbunden sind.

3. Implantierbare elektrische Leitung (20) gemäß Anspruch 1 oder 2, **dadurch gekennzeichnet, dass** der wenigstens eine helixartig gewendelte elektrische Leiter (26.1) von einem Draht aus einem oder mehreren der folgenden Metalle gebildet ist: Pt, Pt/Ir, MP35N, Elgiloy, Edelstahl, Kupfer, Molybdän, Tantal, Gold, Zirkonium, eisenhaltige Legierungen, Tantal-Wolframlegierungen, Refraktaermetalle wie Niob oder Titan, Silber, Palladium, leitfähige Polymere oder Polykondensate oder Kombinationen in einem Mantel/Kern-Draht.

4. Implantierbare elektrische Leitung (20) gemäß wenigstens einem der Ansprüche 1 bis 3, **dadurch gekennzeichnet, dass** die Fasern (50) aus einem oder mehreren der folgenden Metalle oder Kunststoffe gebildet sind: Pt, Pt/Ir, MP35N, Elgiloy, Edelstahl, Kupfer, Molybdän, Tantal, Kupfer, Gold, Zirkonium, eisenhaltige Legierungen, Silber oder Kombinationen in einem Mantel/Kern-Draht sowie Peek, Polyamid, Polyurethan, Carbonfasern, Polyethylen, Polyimid, Aramid, PTFE, ETFE, Spinnengewebe, Spinnenfasern, künstliche Spinnenseide.

5. Implantierbare elektrische Leitung (20) gemäß wenigstens einem der Ansprüche 1 bis 4, **dadurch gekennzeichnet, dass** der wenigstens eine helixartig gewendelte elektrische Leiter (26.1) durch eine Beschichtung isoliert ist.

6. Implantierbare elektrische Leitung (20) gemäß wenigstens einem der Ansprüche 1 bis 5, **dadurch gekennzeichnet, dass** die Fasern (50) durch eine Beschichtung isoliert sind.

7. Implantierbare elektrische Leitung (20) gemäß wenigstens einem der Ansprüche 1 bis 6, **dadurch gekennzeichnet, dass** zwischen dem wenigstens einen helixartig gewendelten elektrischen Leiter (26.1) und den Fasern (50) eine Isolation angeordnet ist.

8. Implantierbare elektrische Leitung (20) gemäß wenigstens einem der Ansprüche 2 bis 7, **dadurch gekennzeichnet, dass** wenigstens eine der Hülsen (40.1; 40.2) mit einer zu dieser konzentrischen, von der Hülse durch ein Dielektrikum elektrisch isolierten Wendelhülse (52) eine Kapazität bildet.

9. Implantierbare elektrische Leitung (20) gemäß wenigstens einem der Ansprüche 1 bis 8, **dadurch gekennzeichnet, dass** das von den Fasern (50) gebildete Fasergeflecht sowohl elektrisch nicht-leitende Fasern aus Kunststoff als auch elektrisch leitenden Fasern aus Metall enthält.

10. Implantierbare elektrische Leitung (20) gemäß wenigstens einem der Ansprüche 1 bis 9, **dadurch gekennzeichnet, dass** die implantierbare elektrische Leitung eine Zuleitung aufweist, die in einem Abschnitt gemäß einem der Ansprüche 1 bis 9 ausgebildet ist.

11. Implantierbare elektrische Leitung (20) gemäß wenigstens einem der Ansprüche 1 bis 10, **dadurch gekennzeichnet, dass** die implantierbare elektrische Leitung (20) eine Herzschrittmacher- und/oder Defibrillator-Elektrodenleitung ist und einen oder mehrere Elektrodenpole aufweist, die mit der Zuleitung elektrisch verbunden sind.

12. Implantierbare elektrische Leitung (20) gemäß wenigstens einem der Ansprüche 1 bis 11, **dadurch gekennzeichnet, dass** die Zuleitung so ausgebildet ist, dass der wenigstens eine helixartig gewendelte elektrische Leiter (26.1) und das Fasergeflecht Induktivitäten (44) und/oder Kapazitäten (46) bilden, die einen Filter bilden, der bei Frequenzen der in Kernspintomografen herrschenden elektromagnetischen Felder sperrt.
